# EUROPEAN PATENT SPECIFICATION

(11) **EP 4 482 391 B1**
(45) Date of publication and mention of the grant of the patent: **11.03.2026**
(21) Application number: 23711145.5
(22) Date of filing: 19.02.2023
(51) Int. Cl.: A61B 5/339, A61B 18/14, A61B 18/00, A61B 5/361, A61B 5/287, A61B 5/06, A61B 5/00, A61B 5/367, A61B 18/12, A61B 17/00, A61B 34/20

(54) **AUTOMATIC STORAGE AND DISPLAY OF ECG SIGNALS INDICATIVE OF ATRIAL FIBRILLATION**
AUTOMATISCHE SPEICHERUNG UND ANZEIGE VON EKG-SIGNALEN, DIE VORHOFFLIMMERN ANZEIGEN
STOCKAGE ET AFFICHAGE AUTOMATIQUES DE SIGNAUX ECG INDICATIFS D'UNE FIBRILLATION AURICULAIRE

(30) Priority: 22.02.2022 US 202217677687
(43) Date of publication of application: 01.01.2025
(73) Proprietor: Biosense Webster (Israel) Ltd., 2066717 Yokneam (IL)
(72) Inventor: TURGEMAN, Aharon, 2066717 Yokneam (IL); COHEN, Benjamin, 2066717 Yokneam (IL); DVORKIN, Vladimir, 2066717 Yokneam (IL); KATZ, Natan Sharon, 2066717 Yokneam (IL)
(74) Representative: Carpmaels & Ransford LLP
(86) International application number: PCT/IB2023/051505
(87) International publication number: WO 2023/161779

(56) References cited:
- US-A1- 2011 066 202
- US-A1- 2020 060 567
- CHOUDRY SUBBARAO ET AL: "RADAR : A Multicenter Food and Drug Administration Investigational Device Exemption Clinical Trial of Persistent Atrial Fibrillation", CIRCULATION: ARRHYTHMIA AND ELECTROPHYSIOLOGY, 16 January 2020 (2020-01-16), pages 2 - 12, XP093043905, Retrieved from the Internet <URL:https://www.ncbi.nlm.nih.gov/pmc/articles/PMC6970579/pdf/hae-13-e007825.pdf> [retrieved on 20230503], DOI: 10.1161/CIRCEP.119.007825

## Description

### FIELD OF THE DISCLOSURE

The present disclosure relates generally to medical devices, and particularly to a system for automatically storing ECG signals indicative of Atrial Fibrillation.

### BACKGROUND OF THE DISCLOSURE

Various techniques for determining arrhythmias, such as Atrial Fibrillation, have been published.

For example, U.S. Patent No. 10,939,863 describes a method that includes receiving, in a processor, a two-dimensional (2D) electro-anatomical (EA) map of an interior surface of at least a portion of a cavity of an organ of a patient, the 2D EA map including electrophysiological (EP) values measured at respective locations on the interior surface. A complex analytic function is fitted to a set of the EP values that were measured in a given region of the 2D EA map. A singularity is identified in the fitted complex analytic function. The region is projected onto a three-dimensional (3D) EA map of the interior surface. At least part of the 3D EA map is presented to a user, including indicating an arrhythmogenic EP activity at a location on the 3D EA map corresponding to the singularity identified in the fitted complex analytic function.

In US2011066202A1, there is described a method that includes selecting an electrode located in a patient wherein the electrode comprises a lead-based electrode; acquiring position information with respect to time for the electrode, during both loaded and unloaded conditions of the lead, where the acquiring uses the electrode for repeatedly measuring electrical potentials in an electrical localization field established in the patient; calculating a both loaded and unloaded stability metrics for the electrode based on the acquired position information with respect to time; and comparing the unloaded and loaded stability metrics to decide whether the electrode, as located in the patient, comprises a stable location for delivery of therapy.

In US2020060567A1, there is described a method including calculating, at multiple intracardiac locations, respective average atrial fibrillation cycle-length (AFCL) values. A determination is made as to whether the calculated average AFCL values are indicative of a regular atrial fibrillation (AF) activity. Gradients between pairs of the average AFCL values are calculated for a plurality of average AFCL values that are determined to be indicative of regular AF activity.

In *"*RADAR: A Multicenter Food and Drug Administration Investigation Device Exemption Clinical Trian of Persistent Atrial Fibrillation", Choudry Subbarao et al, 16.01.2020, XP093043905, there is described a study using a novel system for real-time, high resolution identification of atrial fibrillation (AF) drivers in persistent AF.

### BRIEF DESCRIPTION OF THE DRAWINGS

The present disclosure will be more fully understood from the following detailed description of the examples thereof, taken together with the drawings in which:
Fig. 1 is a schematic, pictorial illustration of a catheter-based position-tracking and ablation system, in accordance with an example of the present disclosure;
Fig. 2 is a schematic, pictorial illustration of a section of a heart, and electrocardiogram (ECG) signals, which are sensed in the section and are indicative of Atrial Fibrillation (AF) in the heart, in accordance with an example of the present disclosure; and
Fig. 3 is a flow chart that schematically illustrates a method for automatically storing and presenting ECG signals indicative of AF, in accordance with an example of the present disclosure.

### DETAILED DESCRIPTION OF EXAMPLES

### OVERVIEW

Examples of the present disclosure that are described hereinbelow provide improved techniques for storing and presenting electrocardiogram (ECG) signals indicative of Atrial Fibrillation in a heart of a patient.

In some examples, a system comprises a catheter having a distal-end assembly (DEA) comprising multiple (e.g., about 48) electrodes arranged in an array for covering a section in the heart. The system further comprises patch electrodes attached to the skin of the chest of the patient, and a magnetic-based position sensor configured to produce position signals indicative of the position (in a predefined coordinate system) of the DEA in the patient heart. When placed in contact with tissue of the heart, each of the electrodes is configured to produce impedance-based position signals indicative of the position of the respective electrode in the predefined coordinate system. Note that the impedance-based position signals are produced based on impedance measurements between (i) each of the electrodes of the DEA, and (ii) one or more of the patch electrodes.

In some examples, when placed in contact with the tissue, each of the electrodes of the DEA is configured to produce electrocardiogram (ECG) signals sensed in the tissue.

In some examples, the system comprises a processor, which is configured to receive during a predefined time interval, for each electrode of the catheter that is placed in contact with tissue: (i) position signals indicative of the position of the electrode, and (ii) the ECG signals acquired by each of the electrodes, as described above.

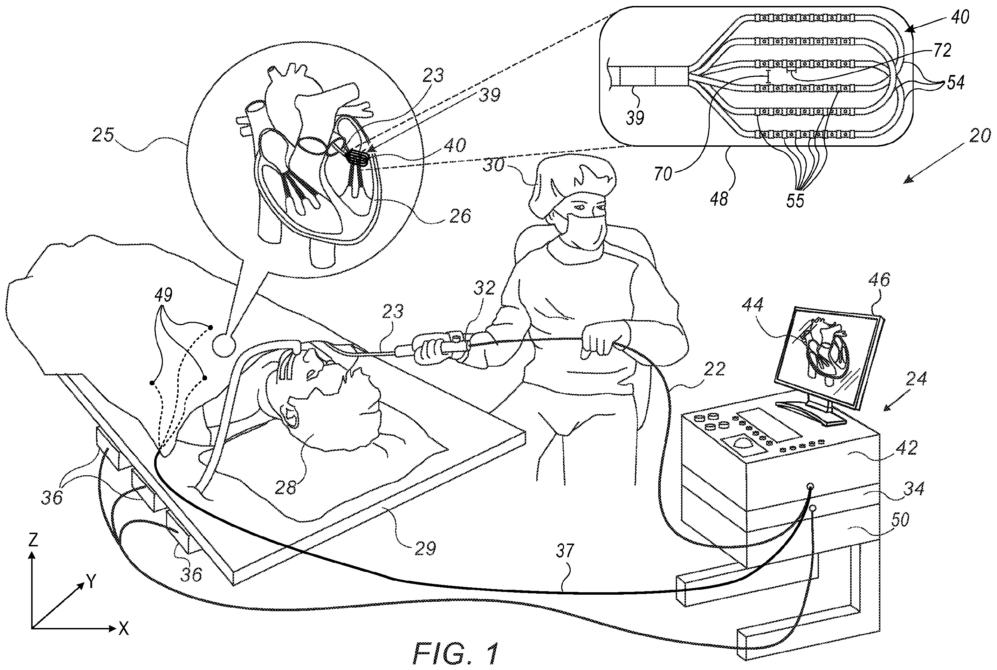

The processor is configured to calculate, for each of the electrodes based on the position signals, a positioning stability along the predefined time interval. The positioning stability may be calculated using an Advanced Catheter Location (ACL) catheter-position tracking method described in detail in Fig. 1 below. In the present example, the impedance-based position signals are used in the ACL, and a standard deviation (SD) of the impedance-based position signals is calculated along the predefined time interval, e.g., the duration of the time interval may be about 2.5 seconds. The processor is configured to hold a threshold indicative of the positioning stability of each electrode of the DEA. For example, the threshold may have a value of about 3 mm, so that electrodes of the DEA whose position signals have a SD smaller than about 3 mm are referred to herein as qualified electrodes, and electrodes of the DEA whose position signals have a SD larger than about 3 mm are referred to as herein as disqualified electrodes.

In some examples, the processor is configured to receive, during the predefined time interval, ECG signals sensed and acquired by the electrodes of the DEA,

In some examples, the processor is configured to calculate, for the qualified electrodes, i.e., for the electrodes whose positioning stability has an error (e.g., SD) smaller than the given threshold (e.g., of 3 mm), whether the ECG signals are indicative of an atrial fibrillation (AF) in the heart.

In some examples, the system comprises a memory, and the processor is configured to control the memory to automatically store the ECG signals that have been identified as indicative of the AF. Note that the ECG signals may be indicative of different attributed indicative of the AF.

In some examples, the system comprises a display, and the processor is configured to control the display to display the stored ECG signals on one or more maps of the heart. The stored ECG signals may be displayed on a single map, such that the ECG signals indicative of a first attribute of the indicated AF are marked using a first tag, and the ECG signals indicative of a second attribute (different from the first attribute) of the indicated AF are marked using a second tag, different from the first tag. In alternative examples, the processor is configured to display on the display: (i) a first map of at least a section of the heart having the ECG signals indicative of the first attribute, and (ii) a second map of at least the section of the heart having the ECG signals indicative of the second attribute. In other words, the ECG signals may be presented on different maps of the heart.

The disclosed techniques improve the quality of electrophysiological (EP) mapping by eliminating ECG signals acquired by electrodes whose positioning stability is insufficient. Moreover, the disclosed techniques reduce the duration of EP mapping procedures by (i) using multi-electrode catheters covering sections in the tissue in question and concurrently acquiring the position signals and the ECG signals using the electrodes of the catheter, and (ii) automating the storage of the ECG signals indicative of Atrial Fibrillation in the heart and the display of the respective ECG signal on one or more maps of the heart.

The invention is defined by the appended claims.

### SYSTEM DESCRIPTION

Fig. 1 is a schematic, pictorial illustration of a catheter-based position-tracking and ablation system 20, in accordance with an example of the present disclosure.

In some examples, system 20 comprises a catheter 22, in the present example a multi-spline and multi-electrode cardiac catheter described below, and a control console 24. In the example described herein, catheter 22 may be used for any suitable therapeutic and/or diagnostic purposes, such as but not limited to sensing of electro-anatomical (EA) information in tissue in question and applying ablation signals to tissue of a heart 26. In the context of the present disclosure, the term information refers to the spatial location of each electrode of the catheter distal end, and an electrocardiogram (ECG) signal sensed by the respective electrodes of catheter 22.

In some examples, console 24 comprises a processor 42, typically a general-purpose computer, with suitable front end and interface circuits for receiving signals from catheter 22 and for controlling other components of system 20 described herein. Processor 42 may be programmed in software to carry out the functions that are used by the system, and is configured to store data for the software in a memory 50. The software may be downloaded to console 24 in electronic form, over a network, for example, or it may be provided on non-transitory tangible media, such as optical, magnetic or electronic memory media. Alternatively, some or all of the functions of processor 42 may be carried out using an application-specific integrated circuit (ASIC) or any suitable type of programmable digital hardware components.

Reference is now made to an inset 25. In some examples, catheter 22 comprises a distal-end assembly (DEA) 40 having multiple (e.g., six) splines, each of which comprises multiple (e.g., eight) electrodes configured to sense signals from tissue of heart 26.

Reference is now made to an inset 48 showing DEA 40. In the present example, DEA 40 comprises a Picasso^{™} catheter, produced by Biosense Webster Inc. (Irvine, Calif.). The Picasso^{™} catheter comprises six splines 54 arranged at a distance 70 from one another, so as to cover a surface in tissue of heart 26. Note that splines 54 are flexible to conform with the tissue in question. Each spline 54 has eight electrodes 55 that, when placed in contact with tissue of heart 26, are configured to produced signals indicative of: (i) electrocardiogram (ECG) signals in the respective tissue, and (ii) impedance, which is indicative of the position of each electrode in an XYZ coordinate system of system 20, as will be described in detail below.

In the present example, electrodes 55 are positioned at a distance 72 from one another. The ECG signals and the sensed impedance may comprise unipolar signals or bipolar signals as will be described hereinafter. In other examples, DEA 40 may comprise any other suitable type of DEA having multiple electrodes arranged in an array that covers a suitable area of tissue of heart 26.

Reference is now made back to the general view of Fig. 1. In some examples, catheter 22 comprises a shaft 23 for inserting DEA 40 to a target location for ablating tissue in heart 26. During an Electrophysiology (EP) mapping and/or ablation procedure, physician 30 inserts catheter 22 through the vasculature system of a patient 28 lying on a table 29. Physician 30 moves DEA 40 to the target location in heart 26 using a manipulator 32 near a proximal end of catheter 22, which is connected to interface circuitry of processor 42. In the present example, the target location may comprise tissue having one or more sites intended to be diagnosed (and optionally ablated) by DEA 40.

In some examples, system 20 comprises external patch electrodes 49, which are coupled to the skin of the chest of patient 28, and are configured to sense signals indicative of ECG and/or impedance.

In some examples, based on the signals received from electrodes 55 (of DEA 40) and external patch electrodes 49, processor 42 is configured to produce position signals, indicative of the position of each electrode 55 in the XYZ coordinate system of heart 26. The position signals are produced using an Advanced Catheter Location (ACL) catheter-position tracking method described below. In the present example, processor 42 is connected to patch electrodes 49, using electrical wires running through a cable 37.

In some examples, processor 42 is configured to determine the position coordinates of each electrode 55, based on impedances measured between each electrode 55 and each of patch electrodes 49. The ACL method of electrode position sensing using system 20 is implemented in various medical applications, for example in the CARTO^{™} system, produced by Biosense Webster Inc. (Irvine, California) and is described in detail in U.S. Patent Nos. 7,756,576, 7,869,865, and 7,848,787.

Reference is now made back to inset 48. In some examples, catheter 22 comprises a position sensor 39 of a magnetic-based position tracking system, which is coupled to the distal end of catheter 22, e.g., in close proximity to DEA 40. In the present example, position sensor 39 comprises a magnetic position sensor, but in other examples, any other suitable type of position sensor (e.g., other than magnetic based) may be used.

Reference is now made back to the general view of Fig. 1. In some examples, during the navigation of DEA 40 in heart 26, processor 42 receives signals from magnetic position sensor 39 in response to magnetic fields from external field generators 36, for example, for the purpose of measuring the position of DEA 40 in heart 26. In some examples, console 24 comprises a driver circuit 34, configured to drive magnetic field generators 36. Magnetic field generators 36 are placed at known positions external to patient 28, e.g., below table 29.

In some examples, processor 42 is configured to display, e.g., on a display 46 of console 24, the tracked position of DEA 40 overlaid on an image 44 of heart 26, which is typically a three-dimensional (3D) image.

The method of position sensing using external magnetic fields is implemented in various medical applications, for example, in the CARTO^{™} system, produced by Biosense Webster Inc. (Irvine, Calif.) and is described in detail in U.S. Patent Nos. 5,391,199, 6,690,963, 6,484,118, 6,239,724, 6,618,612 and 6,332,089, in PCT Patent Publication WO 96/05768, and in U.S. Patent Application Publication Nos. 2002/0065455 A1, 2003/0120150 A1 and 2004/0068178 A1.

In some examples, processor 42 is configured to use the magnetic-based and the ACL-based position signals to calculate, for example, a roll angle of DEA 40, so as to correct the ACL-based position(s) of one or more electrode 55. Moreover, based on the magnetic-based position signals, processor 42 is configured to adjust the orientation of (the flat array of) DEA 40, relative to the tissue in question of heart 26.

One implementation of using a combination of the magnetic-based position tracking system for improving the position sensing performance of an ACL system is described in U.S. Patent Application Publication 2019/0021789, whose inventors are Gliner et al., and is assigned to the applicant of the present disclosure.

In some examples, the ECG signals may comprise: (i) bipolar ECG signals sensed between two electrodes 55 (or between two groups of electrodes 55 or using any other suitable two poles using a suitable arrangement of electrodes 55), or (ii) unipolar signals sensed between each electrode 55 and one or more of patch electrodes 49.

This particular configuration of system 20 is shown by way of example, in order to illustrate certain problems that are addressed by examples of the present disclosure and to demonstrate the application of these examples in enhancing the performance of such a system. Examples of the present disclosure, however, are by no means limited to this specific sort of example system, and the principles described herein may similarly be applied to other sorts of medical systems.

### AUTOMATICALLY STORING AND DISPLAYING ECG SIGNALS INDICATIVE OF ATRIAL FIBRILLATION

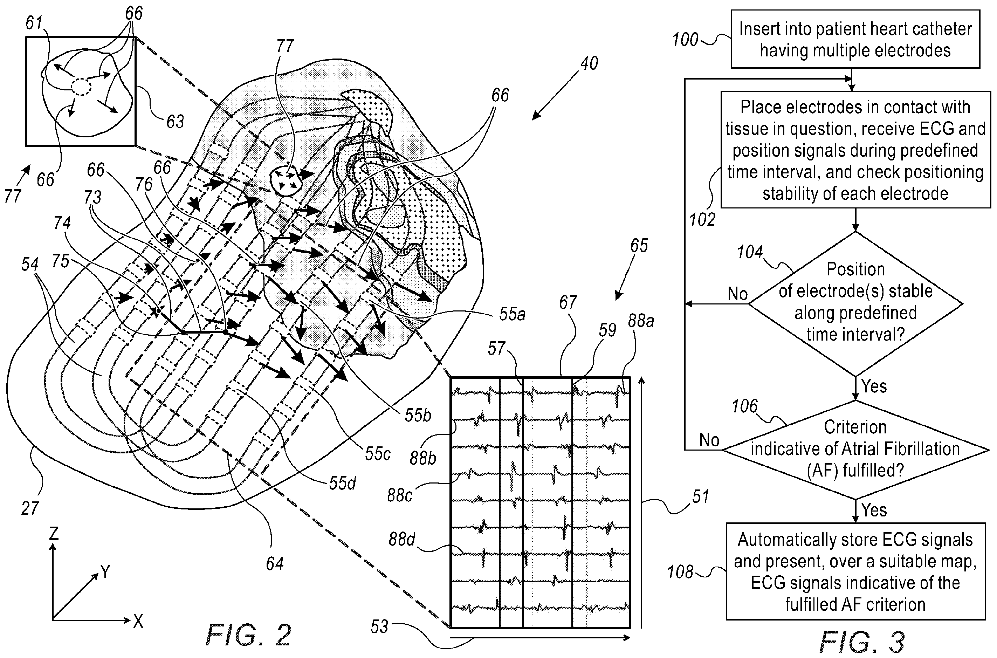

Fig. 2 is a schematic, pictorial illustration of a section 27 of heart 26 (Fig. 1), and electrocardiogram (ECG) signals, which are sensed in section 27 by electrodes 55 of DEA 40, and are indicative of an Atrial Fibrillation (AF) in heart 26, in accordance with an example of the present disclosure.

In some examples, as described in Fig. 1 above, physician 30 moves DEA 40 to the tissue in question, in the present example, section 27 located on an inner wall of an atrium of heart 26. In some examples, splines 54 are placed over section 27, so that at least some of, and typically all 48 electrodes 55, are placed in contact with the tissue of section 27.

In some examples, processor 42 is configured to receive, during a predefined time interval (e.g., between about 1 second and 10 seconds), multiple position signals from each of position sensor 39 and the ACL position tracking system, as described in Fig. 1 above. In the present example, the time interval may comprise about 2.5 seconds, and during this time interval, processor 42 may receive about 150 sets of position signals (i.e., approximately every 16.7 milliseconds). Based on the approximately 150 sets of position signals received during the time interval, processor 42 is configured to estimate the position of each electrode 55 (and of DEA 40) along the time interval. Moreover, processor 42 is configured to calculate, for each electrode 55, the average position and the standard deviation (SD) of the position, along the 2.5-second time interval.

In some examples, processor 42 is configured to hold a threshold indicative of the positioning stability of each electrode 55 along the time interval. In the context of the present disclosure and in the claims, the term "positioning stability along the time interval" refers to the deviations of the calculated position of a given electrode 55 relative to the average position of the given electrode 55 that is calculated based on the position signals received during the predefined time interval (e.g., 2.5 seconds).

In the present example, the threshold has a value of about 3 mm, so that in case the SD of the position, along the 2.5-second time interval, is smaller than 3, the ECG signals received from the given electrode 55 can be used for detecting whether the sensed ECG signals are indicative of an AF in heart 26. Such ECG signals are also referred to herein as qualified ECG signals. In case the SD is larger than about 3 mm, the ECG signals received from the given electrode 55 cannot be used for detecting whether the ECG signals, sensed by the given electrode 55, are indicative of the AF in heart 26. Such ECG signals are also referred to herein as disqualified ECG signals.

In some examples, in case an insufficient number of electrodes (e.g., less than 3 electrodes) whose SD is smaller than 3 mm is identified, physician 30 define a new time interval (having the same duration of about 2.5 seconds, or a different duration) for repeating the collection of the position signals and ECG signals from the tissue in section 27 of heart 26.

In the context of the present disclosure and in the claims, the terms "about" or "approximately" for any numerical values or range of values indicate a suitable dimensional tolerance that allows (i) the part or collection of components, and (ii) a measurable abstract feature, such as the aforementioned time interval and measured values related to ECG and position signals, to function for its intended purpose as described herein.

In some examples, after obtaining ECG measurements from a sufficient number of (e.g., 3 or more) electrodes 55, processor 42 is configured to calculate several features and/or attributes and/or parameters indicative of whether the ECG signals are indicative of the AF in heart 26. Note that ECG signals sensed by electrodes 55 whose SD is larger than 3, are not qualified for providing an indication of AF, and therefore, processor 42 is configured to filter out these ECG signals from the calculation of the features and/or attributes and/or parameters mentioned above.

In the context of the present disclosure and in the claims, the terms "feature," "attribute," "parameter," and "criterion," and grammatical variations thereof, are used interchangeably and refer to an indication of whether the ECG signals described above, are indicative of the AF in heart 26.

Note that Atrial Fibrillation causes altering of the calculated parameters over time. Therefore, it is important to select parameters that can be indicative of the AF. For example, atrial fibrillation cycle-length (AFCL) may be indicative of AF in heart 26. In the present example, AFLCs having a value between about 120 and 200 milliseconds (ms) are indicative of a healthy area in heart 26, whereas AFCL values smaller than about 120 ms or larger than about 200 ms, are indicative of a problem that may be related to AF in heart 26. More specifically, a gradient of the AFCL values along different locations along tissue of an atrium, may be used for mapping AF in the respective atrium. Such techniques are described, for example, in U.S. Patent Application Serial No. 11,160,481 assigned to the present applicant. Note that in case of insufficient positioning stability (e.g., having a SD larger than about 3 mm) of one or more electrodes used for sensing the ECG signals, may result in insufficient accuracy of the calculated AFCL values and gradient.

In some examples, processor 42 is configured to apply various types of filters to ECG signals received from electrodes 55 whose SD is smaller than 3 mm, so as to calculate whether the qualified ECG signals are indicative of an AF in heart 26. In some examples, in response to identifying qualified ECG signals that are used for calculating parameters indicative of AF, processor 42 is configured to automatically store these ECG signals, e.g., in memory 50 of console 24.

In some examples, based on the stored ECG signals, processor 42 is configured to identify indications of AF in section 27 of heart 26. In the example of Fig. 2, processor 42 is configured to display on a 3D anatomical map of section 27, conduction velocity vectors 66 indicative of the propagation direction and speed of electrophysiological (EP) signals, e.g., on the surface of the tissue of section 27.

In some examples, processor 42 is configured to identify patterns indicative of AF. In the example of Fig. 2, processor 42 is configured to identify, within section 27, a section 77 having a focal point 61 in the tissue of heart 26. Reference is now made to an inset 63. In some examples, conduction velocity vectors 66 are arranged as if they are being radiated from focal point 61. More specifically, focal point 61 is a virtual point or area, which is the origin of vectors 66, as shown in inset 63. In such examples, processor 42 may apply various techniques of pattern recognition for identifying focal point 61 in section 77.

Reference is now made back to the general view of Fig. 2. In some examples, in case three points located within a subsection of section 27 have a gradient of the AFCL value, this subsection may be indicative of AF. In the example of Fig. 2, based on the qualified ECG signals, processor 42 has calculated along lines 73 AFCL values of about 150 ms, 200 ms and 270 ms at points 74, 75 and 76, respectively. This gradient of the AFCL values is indicative of a potential AF, and therefore, processor 42 is configured to identify and store the ECG signals measured by electrodes 55 in the respective subsection that is in close proximity with lines 73. Moreover, processor 42 is configured to display over the map of section 27, lines 73 or any other indication of the AFCL gradient.

Reference is now made to a subsection 64 of the map of section 27. In some examples, some of electrodes 55 are positioned within the area of subsection 64, more specifically, electrodes 55a, 55b, 55c and 55d are among these electrodes.

Reference is now made to an inset 67, showing a group of graphs 65, which presents a dispersion of sequential activations of consecutive bipolar ECG signals produced by electrodes 55 located within subsection 64. For example, graphs 88a, 88b, 88c and 88d are produced by electrodes 55a, 55b, 55c and 55d, respectively. The amplitude of the bipolar signals is presented along an axis 51, and the amplitude of the signal over time is presented along an axis 53. Moreover, the time interval of the graphs is spanning 100% of the atrial fibrillation cycle length described above.

In the present example, group of graphs 65 comprises vertical markers 57 and 59. As shown in inset 67, the peaks (e.g., R-peaks) indicative of the activation in the respective ECG signals are located at an offset relative to one another along axis 53. For example, a peak of graph 88a falls on marker 59, whereas the positions of peaks of graphs 88b, 88c and 88d are shifted, and therefore, the peaks are not falling on marker 59. In another example, the peaks of graphs 88b and 88c precede the time point of marker 57, and the peak of graphs 88a and 88d are later from the time point of marker 57. This dispersion of sequential activations of the consecutive bipolar ECG signals may be indicative of AF source.

In other examples, processor 42 is configured to identify other parameters and/or features and/or attributes indicative of AF, for example, using any suitable calculation or manipulation on qualified ECG signals, as described above.

Fig. 3 is a is a flow chart that schematically illustrates a method for automatically storing and presenting ECG signals indicative of AF in heart 26, in accordance with an example of the present disclosure.

The method begins at a catheter inserting step 100, with physician 30 inserting DEA 40 (of catheter 22) having multiple (e.g., 48) electrodes 55, as described in Fig. 1 above. At a positioning stability checking step 102, physician 30 places electrodes 55 in contact with the tissue in question of section 27 in heart 26. In some examples, processor 42 receives during a predefined time interval, e.g., of about 2.5 seconds: (i) position signals from electrodes 55 and patch electrodes 49, and (ii) ECG signals from electrodes 55. Moreover, based on the received position signals, processor 42 checks the positioning stability of each electrode 55, as described in detail in Figs. 1 and 2 above.

At a first decision step 104, processor 42 compares between: (i) a threshold having a predefined value (e.g., about 3 mm), and (ii) for each electrode 55, the calculated SD of the position signals received along the time interval, as described in Fig. 2 above. In case the calculated SD is larger than the threshold, the method loops back to step 102.

In some examples, processor 42 also defines a minimal number of qualified electrodes 55, i.e., electrodes 55 whose positioning stability has an error (i.e., SD) smaller than the threshold. In case the number of qualified electrodes 55 is smaller than the minimal number, the method loops back to step 102. For example, in case only one or two electrodes 55 (e.g., out of the 48 electrodes of DEA 40) have the calculated SD smaller than about 3 mm, the method loops back to step 102.

In case the calculated SD of the position signals for a given electrode 55 is smaller than the 3-mm threshold, the given electrode 55 is qualified, and the ECG signals produced by the given electrode 55 during the same 2.5-second time interval, will be used by processor 42 in later steps of the method.

In some examples, in case the number of qualified electrodes is larger than the minimal number defined in processor 42, the method proceeds to a second decision step 106. Note that in step 106, only the ECG signals of the qualified electrodes are used. In step 106, processor 42 is configured to calculate whether the ECG signals produced during the time interval by the qualified electrodes 55, are indicative of an atrial fibrillation (AF) in heart 26. In other words, processor 42 is configured to check whether a criterion indicative if the AF is fulfilled based on the ECG signals produced during the time interval by the qualified electrodes 55. For example, processor 42 may apply various techniques of pattern recognition for identifying focal point 61 in section 77, as described in Fig. 2 above.

In case processor 42 identifies that the ECG signals of step 106 above are indicative of AF, the method proceeds to an automatic storage step 108. In step 108 that concludes the method, processor 42 automatically stores, in memory 50, the ECG signals used for calculating parameters indicative of the AF, as described in Fig. 2 above. In some examples, processor 42 is configured to visualize, the criterion indicative of the AF. over a suitable anatomical or electro-anatomical map of heart 26. The visualized criterion is based on a calculation of the ECG signals that are indicative of the AF in heart 26, as described in Fig. 2 above.

In some examples, in case processor 42 does not identify that the ECG signals of step 106 above are indicative of AF, the method loops back to step 102, and physician 30 moves DEA 40 to cover another section, other than section 27, in heart 26. Note that in the other section, processor 42 and/or physician 30 may select the duration of the time interval to be similar to the time interval selected when electrodes 55 of DEA 40 were placed in contact with section 27, e.g., 2.5 seconds. In alternative examples, processor 42 and/or physician 30 may select a different duration of the time interval for sensing the position signals and ECG signals in the other section.

In some examples, the disclosed technique provides physician 30 with automatic storage of ECG signals that are: (i) obtained in a stable positioning of the respective electrode(s) 55, (ii) obtained in a relatively large section (e.g., section 27) of heart 26 covered by DEA 40 during a short time interval (e.g., about 2.5 seconds), and (iii) indicative of AF in the heart 26. In principle, it is possible to acquire ECG signals by visiting point by in section 27, but this process may prolong the procedure substantially, and the points are not acquired at the same time interval, so the results may not be representative of the AF compared with the results obtained using the techniques described in Fig. 2 above and in the method of Fig. 3.

In other examples, in case: (i) physician 30 decides to acquire ECG signals only in section 27, or (ii) after repeating steps 102-108 in additional sections in heart 26, the ECG mapping is concluded. In such examples, processor 42 presents the stored ECG signals, which are indicative of AF, over one or more maps of heart 26.

In some examples, at least one of the stored ECG signals may be displayed in a separate map. For example, processor 42 may produce a map of heart 26 having focal points 61 identified at one or more sections of heart 26.

In other examples, processor 42 may display all the ECG signals, which are indicative of AF and are automatically stored in memory 50, in a single map of heart 26, as shown for example in the map of section 27 of Fig. 2 above. In such examples, processor 42 is configured to add different tags to ECG signals included in different attributes of the AF. For examples, (i) section 77 indicative of focal point 61, and (ii) lines 73 indicative of the AFCL gradient along points 74-76, are bot shown on the map of section 27 using different graphic representation and/or tagging, as shown and described in Fig. 2 above.

The method of Fig. 3 is simplified, and is provided by way of example for the sake of conceptual clarity. In other examples, Fig. 3 may comprise additional or alternative steps that, for example, are using different implementation or order of steps, in accordance with the techniques disclosed in Figs. 1-3 above.

The examples described herein mainly address automatically storing and presenting ECG signals indicative of Atrial Fibrillation in a heart.

## Claims

1. A system (20), comprising:
a processor (42), which is configured to:
receive during a predefined time interval, for each electrode (55) of a catheter (22) having multiple electrodes that are placed in contact with tissue of a heart of a patient: (i) position signals indicative of a position of the electrode, and (ii) electrocardiogram, ECG, signals acquired by the electrode;
calculate, for each of the electrodes based on the position signals, a positioning stability over the predefined time interval, wherein the processor is configured to calculate the positioning stability for each electrode by calculating, using the position signals received from the electrode over the predefined time interval, a standard deviation, SD, of the position signals for
the electrode, wherein the standard deviation is calculated relative to an average position of the electrode calculated based on the position signals received during the predefined time interval;
compare, for each electrode, the calculated SD of the position signals received over the time interval, with a threshold having a predefined value, to determine a number of qualified electrodes having an SD smaller than the threshold; and
only if the number of qualified electrodes is larger than a minimum number defined by the processor, calculate, for the qualified electrodes, whether the ECG signals are indicative of an atrial fibrillation, AF, in the heart; and
a memory (50), which is configured to store the ECG signals that are indicative of the AF.

2. The system according to claim 1, wherein the processor is configured to store in the memory: (i) a first set of the ECG signals indicative of a first attribute of the AF, and (ii) a second set of the ECG signals, different from the first set, indicative of a second attribute of the AF, wherein the second attribute is different from the first attribute.

3. The system according to claim 2, comprising a display (46), which is configured to display the stored ECG signals on one or more maps of the heart.

4. The system according to claim 3, wherein the processor is configured to assign:
(i) a first graphic representation to the first set of ECG signals and to the first attribute, and (ii) a second graphic representation to the second set of ECG signals and to the second attribute, and wherein the display is configured to display one or both of: (i) the first and second sets, and (ii) the first and second attributes, on the one map of the heart.

5. The system according to claim 3, wherein the processor is configured to assign:
(i) a first graphic representation to the first set of ECG signals and to the first attribute, and (ii) a second graphic representation to the second set of ECG signals and to the second attribute, and wherein the display is configured to display: (i) one or both of the first set and the first attribute on a first map of the heart, and (ii) one or both of the second set and the second attribute on a second map of the heart, different from the first map.

6. The system according to claim 2, wherein the first attribute comprises a focal point and the second attribute comprises a gradient of an atrial fibrillation cycle length , AFCL.

7. The system according to claim 1, wherein the processor is configured to select the predefined time interval by selecting a duration of the predefined time interval between 1 second and 10 seconds.

8. The system according to claim 7, wherein the processor is configured to repeat, for an additional section of the heart to which the array of the electrodes has been moved and which the electrodes are placed in contact with, the: (a) receiving of the position signals and the ECG signal from each of the electrode over a given time interval, (b) calculating of the positioning stability, over the given time interval, for each of the electrodes, and (c) calculating, for the electrodes whose positioning stability has an error smaller than a given threshold, whether the ECG signals are indicative of the AF, and wherein the memory is configured to repeat the storing of the ECG signals that are indicative of the AF.

9. The system according to claim 8, wherein the tissue is at a first position in the heart and the additional section is at a second position in the heart, different from the first position, and wherein the processor is configured to set a similar duration to (i) the predefined time interval, and (ii) the given time interval.

## Patentansprüche

1. System (20), umfassend:
einen Prozessor (42), der konfiguriert ist zum:
während eines vorgegebenen Zeitintervalls für jede Elektrode (55) eines Katheters (22) mit mehreren Elektroden, die in Kontakt mit Gewebe eines Herzens eines Patienten gebracht sind, Empfangen von: (i) Positionssignalen, die eine Position der Elektrode angeben, und (ii) Elektrokardiogramm-, EKG-Signalen, die von der Elektrode erfasst werden;
Berechnen, für jede der Elektroden basierend auf den Positionssignalen, einer Positionierungsstabilität über das vorgegebene Zeitintervall, wobei der Prozessor konfiguriert ist, um die Positionierungsstabilität für jede Elektrode zu berechnen, durch Berechnen, unter Verwendung der von der Elektrode über das vorgegebene Zeitintervall empfangenen Positionssignale, einer Standardabweichung, SD, der Positionssignale für die Elektrode, wobei die Standardabweichung relativ zu einer durchschnittlichen Position der Elektrode berechnet wird, die basierend auf den während des vorgegebenen Zeitintervalls empfangenen Positionssignalen berechnet wird;
Vergleichen, für jede Elektrode, der berechneten SD der über das Zeitintervall empfangenen Positionssignale mit einem Schwellenwert, der einen vordefinierten Wert aufweist,
um eine Anzahl qualifizierter Elektroden mit einer SD kleiner als der Schwellenwert zu bestimmen; und
nur wenn die Anzahl der qualifizierten Elektroden größer ist als eine vom Prozessor definierte Mindestanzahl, Berechnen, für die qualifizierten Elektroden, ob die EKG-Signale auf ein Vorhofflimmern, AF, im Herzen hinweisen; und einen Speicher (50), der konfiguriert ist, um die EKG-Signale zu speichern, die auf das Vorhofflimmern hinweisen.

2. System nach Anspruch 1, wobei der Prozessor konfiguriert ist, um im Speicher Folgendes zu speichern: (i) einen ersten Satz der EKG-Signale, der auf ein erstes Attribut des AF hinweist, und (ii) einen zweiten Satz der EKG-Signale, der sich von dem ersten Satz unterscheidet und auf ein zweites Attribut des AF hinweist, wobei sich das zweite Attribut von dem ersten Attribut unterscheidet.

3. System nach Anspruch 2, umfassend eine Anzeige (46), die konfiguriert ist, um die gespeicherten EKG-Signale auf einer oder mehreren Karten des Herzens anzuzeigen.

4. System nach Anspruch 3, wobei der Prozessor konfiguriert ist zum Zuweisen von: (i) einer ersten grafischen Darstellung zu dem ersten Satz von EKG-Signalen und zu dem ersten Attribut, und (ii) einer zweiten grafischen Darstellung zu dem zweiten Satz von EKG-Signalen und zu dem zweiten Attribut, und wobei die Anzeige konfiguriert ist, um eines oder beide anzuzeigen von: (i) dem ersten und dem zweiten Satz und (ii) dem ersten und dem zweiten Attribut auf der einen Karte des Herzens.

5. System nach Anspruch 3, wobei der Prozessor konfiguriert ist zum Zuweisen von: (i) einer ersten grafischen Darstellung zu dem ersten Satz von EKG-Signalen und zu dem ersten Attribut, und (ii) einer zweiten grafischen Darstellung zu dem zweiten Satz von EKG-Signalen und zu dem zweiten Attribut, und wobei die Anzeige konfiguriert ist, um Folgendes anzuzeigen: (i) einen oder beide von dem ersten Satz und dem ersten Attribut auf einer ersten Karte des Herzens, und (ii) einen oder beide von dem zweiten Satz und dem zweiten Attribut auf einer zweiten Karte des Herzens, die sich von der ersten Karte unterscheidet.

6. System nach Anspruch 2, wobei das erste Attribut einen Brennpunkt umfasst und das zweite Attribut einen Gradienten einer Vorhofflimmern-Zykluslänge, AFCL, umfasst.

7. System nach Anspruch 1, wobei der Prozessor konfiguriert ist, um das vordefinierte Zeitintervall durch Auswählen einer Dauer des vorgegebenen Zeitintervalls zwischen 1 Sekunde und 10 Sekunden auszuwählen.

8. System nach Anspruch 7, wobei der Prozessor konfiguriert ist, um für einen zusätzlichen Abschnitt des Herzens, zu dem die Anordnung der Elektroden bewegt wurde und mit dem die Elektroden in Kontakt gebracht sind, Folgendes zu wiederholen: (a) Empfangen der Positionssignale und des EKG-Signals von jeder der Elektroden über ein gegebenes Zeitintervall, (b) Berechnen der Positionierungsstabilität über das gegebene Zeitintervall für jede der Elektroden, und (c) Berechnen, für die Elektroden, deren Positionierungsstabilität einen Fehler kleiner als ein gegebener Schwellenwert aufweist, ob die EKG-Signale auf das AF hinweisen, und wobei der Speicher konfiguriert ist, um das Speichern der EKG-Signale, die auf das AF hinweisen, zu wiederholen.

9. System nach Anspruch 8, wobei sich das Gewebe an einer ersten Position im Herzen befindet und sich der zusätzliche Abschnitt an einer zweiten Position im Herzen befindet, die sich von der ersten Position unterscheidet, und wobei der Prozessor konfiguriert ist, um eine ähnliche Dauer für (i) das vordefinierte Zeitintervall und (ii) das gegebene Zeitintervall festzulegen.

## Revendications

1. Système (20), comprenant :
un processeur (42) qui est configuré pour :
recevoir pendant un intervalle de temps prédéfini, pour chaque électrode (55) d'un cathéter (22) ayant de multiples électrodes qui sont placées en contact avec un tissu d'un cœur d'un patient : (i) des signaux de position indiquant une position de l'électrode, et (ii) des signaux d'électrocardiogramme, ECG, acquis par l'électrode ;
calculer, pour chacune des électrodes en fonction des signaux de position, une stabilité de positionnement sur l'intervalle de temps prédéfini, dans lequel le processeur est configuré pour calculer la stabilité de positionnement pour chaque électrode en calculant, à l'aide des signaux de position reçus de l'électrode sur l'intervalle de temps prédéfini, un écart-type, SD, des signaux de position pour l'électrode, dans lequel l'écart-type est calculé par rapport à une position moyenne de l'électrode calculée en fonction des signaux de position reçus pendant l'intervalle de temps prédéfini ;
comparer, pour chaque électrode, le SD calculé des signaux de position reçus sur l'intervalle de temps, à un seuil ayant une valeur prédéfinie,
pour déterminer un nombre d'électrodes qualifiées ayant un SD plus petit que le seuil ; et
uniquement si le nombre d'électrodes qualifiées est plus grand qu'un nombre minimal défini par le processeur, calculer, pour les électrodes qualifiées, si les signaux ECG indiquent une fibrillation auriculaire, AF, dans le cœur ; et une mémoire (50), qui est configurée pour stocker les signaux ECG qui indiquent l'AF.

2. Système selon la revendication 1, dans lequel le processeur est configuré pour stocker dans la mémoire : (i) un premier ensemble des signaux ECG indiquant un premier attribut de l'AF, et (ii) un second ensemble des signaux ECG, différent du premier ensemble, indiquant un second attribut de l'AF, dans lequel le second attribut est différent du premier attribut.

3. Système selon la revendication 2, comprenant un affichage (46), qui est configuré pour afficher les signaux ECG stockés, sur une ou plusieurs cartes du cœur.

4. Système selon la revendication 3, dans lequel le processeur est configuré pour attribuer : (i) une première représentation graphique au premier ensemble de signaux ECG et au premier attribut, et (ii) une seconde représentation graphique au second ensemble de signaux ECG et au second attribut, et dans lequel l'affichage est configuré pour afficher l'un et/ou l'autre parmi : (i) les premier et second ensembles, et (ii) les premier et second attributs, sur la carte précitée du cœur.

5. Système selon la revendication 3, dans lequel le processeur est configuré pour attribuer : (i) une première représentation graphique au premier ensemble de signaux ECG et au premier attribut, et (ii) une seconde représentation graphique au second ensemble de signaux ECG et au second attribut, et dans lequel l'affichage est configuré pour afficher : (i) l'un et/ou l'autre parmi le premier ensemble et le premier attribut sur une première carte du cœur, et (ii) l'un et/ou l'autre parmi le second ensemble et le second attribut sur une seconde carte du cœur, différente de la première carte.

6. Système selon la revendication 2, dans lequel le premier attribut comprend un point focal et le second attribut comprend un gradient d'une longueur de cycle de fibrillation auriculaire, AFCL.

7. Système selon la revendication 1, dans lequel le processeur est configuré pour sélectionner l'intervalle de temps prédéfini en sélectionnant une durée de l'intervalle de temps prédéfini entre 1 seconde et 10 secondes.

8. Système selon la revendication 7, dans lequel le processeur est configuré pour répéter, pour une section supplémentaire du cœur vers laquelle le réseau des électrodes a été déplacé et avec laquelle les électrodes sont placées en contact : (a) la réception des signaux de position et du signal ECG provenant de chacune des électrodes sur un intervalle de temps donné, (b) le calcul de la stabilité de positionnement, sur l'intervalle de temps donné, pour chacune des électrodes, et (c) le fait de calculer, pour les électrodes dont la stabilité de positionnement a une erreur plus petite qu'un seuil donné, si les signaux ECG indiquent l'AF, et dans lequel la mémoire est configurée pour répéter le stockage des signaux ECG qui indiquent l'AF.

9. Système selon la revendication 8, dans lequel le tissu est au niveau d'une première position dans le cœur et la section supplémentaire est au niveau d'une seconde position dans le cœur, différente de la première position, et dans lequel le processeur est configuré pour régler une durée similaire à (i) l'intervalle de temps prédéfini, et à (ii) l'intervalle de temps donné.
